# EUROPEAN PATENT APPLICATION

(11) **EP 2 472 263 A1**
(43) Date of publication of application: **04.07.2012**
(21) Application number: 11305002.5
(22) Date of filing: 03.01.2011
(51) Int. Cl.: G01N 33/574

(54) **Methods for the prognostic assessment of breast cancer**

(71) Applicant: COMMISSARIAT A L'ENERGIE ATOMIQUE ET AUX ENERGIES ALTERNATIVES, 75015 Paris (FR)
(72) Inventor: Filhol-Cochet, Odile, 38640, Claix (FR); Cochet, Claude, 38640, Claix (FR); Charpin, Colette, 13260, Cassis (FR)
(74) Representative: Cabinet Plasseraud

(57) **Abstract**

The present invention relates to a method of determining the prognosis of a patient suffering from breast cancer, comprising the step of measuring the level of expression of the CK2α subunit in breast cancer cells obtained from said patient.

SEQUENCE LISTING

<110>  CEA

<120>  Methods for the prognostic assessment of breast cancer

<130>  BEP101122

<160>  2

<170>  PatentIn version 3.3

<210>  1
<211>  13
<212>  PRT
<213>  Artificial

<220>
<223>  epitope

<400>  1

Val Asn Thr His Arg Pro Arg Glu Tyr Trp Asp Tyr Glu
1               5                   10


<210>  2
<211>  12
<212>  PRT
<213>  Artificial

<220>
<223>  substrate

<400>  2

Arg Arg Arg Glu Asp Glu Glu Ser Asp Asp Glu Glu
1               5                   10

## Description

### FIELD OF THE INVENTION

The present invention relates to methods for the prognostic assessment of breast cancer.

### BACKGROUND OF THE INVENTION

There is a growing consensus that the outcome of patient diagnosed with cancer may be significantly improved by personalized medicine. Thus biomarker discovery from clinically relevant specimens is critically needed to predict response based on underlying molecular biology. Protein kinase-mediated phosphorylation is an important post-translational modification regulating key cellular processes.¹ Tight regulation of phosphorylation events is crucial to the proper function of many cellular signalling pathways and loss of regulation in these pathways underlies many human diseases including cancer.^{1,2}

Protein kinase CK2, formerly referred as to Casein Kinase II, is a multi-subunit enzyme consisting in two catalytic α and α' subunits associated with a dimer of regulatory β subunits.³ CK2 is a multifunctional and pleiotropic ser/thr kinase that plays major roles in cell cycle progression, apoptosis, cell differentiation and transcription processes.¹⁻³ As a signalling protein, CK2 could be targeted to different cellular compartments in response to various stresses such as hypoxia, heat shock, DNA damage.¹⁻³ In addition, CK2α is overexpressed and its activity is enhanced in multiple forms of human cancers⁴ including prostate⁵ and endometrium.⁶ Thus, CK2 is now regarded as a potential target for specific therapy in human malignancies.⁷⁻⁹ This enzyme is also postulated to contribute to breast carcinoma development because enforced overexpression of CK2α in the mammary gland of transgenic mice promotes hyperplasia and neoplasia of the mammary gland.¹⁰ Moreover, an upregulation of CK2 protein and activity was observed during the development of DMBA-induced mammary tumors suggesting a pathologic relationship between CK2 expression and mammary tumorigenesis.¹⁰ Primary breast cancer samples from patients as well as breast cancer cell lines display increased CK2 activity. In addition, a strong CK2α staining, assessed by immunohistochemistry (IHC) in a small series of patients was observed in prostate tumors compared to normal tissue.¹¹

However, no study has shown that CK2α subunit expression in breast carcinoma evaluated, alone or along with other proteins involved in major signalling pathways, could be a prognostic indicator when correlated with patients' outcome.

Furthermore, our goal was also to identify an immunocytochemical signature predictive of poor outcome that would enable to select node negative patients who might benefit from more aggressive therapy and also that could significantly reduce unnecessary treatment.

### SUMMARY AND DETAILED DESCRIPTION OF THE INVENTION

The present invention relates to a method of determining the prognosis of a patient suffering from breast cancer, comprising the step of measuring the level of expression of the CK2α subunit in breast cancer cells obtained from said patient.

A high CK2α expression is associated with a poor prognosis. Indeed, the data shown in the example demonstrate that a lower survival rate is obtained in the group of patients with higher CK2α expression.

The present invention also enables the evaluation of the risk of recurrence of a patient which has been surgically treated.

CK2α levels in patients with metastasis were significantly greater than those with disease free survival. A high CK2α expression is associated with a high-risk of disease recurrence and distant metastasis development.

Typically, a method according to the invention may further comprise the step of comparing the level of expression of CK2α to a threshold level, wherein the subject is prognosed as having a poor prognosis or a high risk of developing metastasis if the CK2α level is significantly higher than the threshold level. Typically the threshold level may be determined by measuring the CK2α level in patients with disease free survival.

In an embodiment of the invention, the patient suffering from breast cancer is a node-negative patient.

Typically said breast cancer cells obtained from said patient originate from a surgical act of tumour resection performed on the patient. In certain other embodiments, said breast cancer cells obtained from said patient originate from a biopsy surgical act wherein a piece of tumour tissue is collected from the patient for further analysis.

In one embodiment of the methods defined above, the levels of expression of 1, 2, 3 or 4 predictive markers are quantified together with the expression of CK2α, wherein the predictive markers are selected from the group consisting of SHARP-2, EIF4E, pMAPK, and pAKT.

In a further embodiment, the levels of expression of 1, 2, 3, 4, 5, 6, 7, or 8 predictive markers are quantified together with the expression of CK2α, wherein the predictive markers are selected from the group consisting of SHARP2, STAT1, EIF4E, pAKT, caveolin, pMAPK, VEGF and FGFR-1.

In a further embodiment, the levels of expression of 1, 2, 3, 4, 5, 6, 7, 8, 9, or 10 predictive markers are quantified in a node-negative patient together with the expression of CK2α, wherein the predictive markers are selected from the group consisting of HIF-1α, SHARP2, EIF4E, pMAPK, MMP7, pAKT, FGFR-1, pmTOR, Fyn and JAK.

Typically, a method of prognosis according to the invention may be used in combination with any other methods already used for the prognostic assessment of breast cancer, including stage, demographic and anthropometric parameters, results of routine clinical or laboratory examination, including size of the tumor, histoprognostic grading, hormone receptors, oncotype, mammoprint, uPA/PAI-1...

By "level of expression" it is meant the level of expression at the protein level.

Typically, the level of expression of the predictive marker may be measured by contacting the sample with a binding partner capable of selectively interacting with the predictive marker, CK2α in particular, in the sample. The binding partner is generally an antibody that may be polyclonal or monoclonal, preferably monoclonal.

In one embodiment, CK2α expression may be measured with an antibody or a fragment thereof disclosed in WO2008083998 which specifically binds to a CK2-α epitope as set forth in SEQ ID NO:1.

The level of the predictive marker(s), CK2α in particular, may also be measured using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the predictive marker and the antibody or antibodies reacted therewith.

The aforementioned assays generally involve separation of unbound protein in a liquid phase from a solid phase support to which antigen-antibody complexes are bound. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like.

More particularly, an ELISA method can be used, wherein the wells of a microtiter plate are coated with antibodies against the predictive marker(s), CK2α in particular. A biological sample containing or suspected of containing the predictive marker(s) is then added to the coated wells. After a period of incubation sufficient to allow the formation of antibody-antigen complexes, the plate(s) can be washed to remove unbound moieties and a detectably labeled secondary binding molecule added. The secondary binding molecule is allowed to react with any captured sample marker protein, the plate washed and the presence of the secondary binding molecule detected using methods well known in the art.

Alternatively an immunohistochemistry (IHC) method may be preferred. IHC specifically provides a method of detecting targets in a sample or tissue specimen in situ. The overall cellular integrity of the sample is maintained in IHC, thus allowing detection of both the presence and location of the targets of interest. Typically a sample is fixed with formalin, embedded in paraffin and cut into sections for staining and subsequent inspection by light microscopy. Current methods of IHC use either direct labeling or secondary antibody-based or hapten-based labeling. Examples of known IHC systems include, for example, EnVision(TM) (DakoCytomation), Powervision(R) (Immunovision, Springdale, AZ), the NBA(TM) kit (Zymed Laboratories Inc., South San Francisco, CA), HistoFine(R) (Nichirei Corp, Tokyo, Japan), BenchMark XT (TM) (Ventana Medical Systems, Inc).

In particular embodiment, a tissue section may be mounted on a slide or other support after incubation with antibodies directed against the predictive marker(s), CK2α in particular. Then, microscopic inspections in the sample mounted on a suitable solid support may be performed. For the production of photomicrographs, sections comprising samples may be mounted on a glass slide or other planar support, to highlight by selective staining the presence of the predictive marker(s), CK2α in particular.

In some embodiments, an IHC staining procedure may comprise steps such as: cutting and trimming tissue, fixation, dehydration, paraffin infiltration, cutting in thin sections, mounting onto glass slides, baking, deparaffination, rehydration, antigen retrieval, blocking steps, applying primary antibodies, washing, applying secondary antibodies (optionally coupled to a suitable detectable label), washing, counter staining, and microscopic examination.

The invention also provides a kit comprising:
a) a binding partner capable of selectively interacting with CK2-α ; and
b) 1, 2, 3, or 4 different binding partners which specifically bind to 1, 2, 3 or 4 respectively, predictive markers selected from the group consisting of SHARP-2, EIF4E, pMAPK, and pAKT.

The invention also provides a kit comprising:
a) a binding partner capable of selectively interacting with CK2-α ; and
b) 1, 2, 3, 4, 5, 6, 7, or 8 different binding partners which specifically bind to 1, 2, 3, 4, 5, 6, 7, or 8 respectively, predictive markers selected from the group consisting of SHARP2, STAT1, EIF4E, pAKT, caveolin, pMAPK, VEGF and FGFR-1.

The invention also provides a kit comprising:
a) a binding partner capable of selectively interacting with CK2-α ; and
b) 1, 2, 3, 4, 5, 6, 7, 8, 9, or 10 different binding partners which specifically bind to 1, 2, 3, 4, 5, 6, 7, 8, 9, or 10 respectively, predictive markers selected from the group consisting of HIF-1α, SHARP2, EIF4E, pMAPK, MMP7, pAKT, FGFR-1, pmTOR, Fyn and JAK.

In one embodiment, the binding partner is an antibody or a fragment thereof.

The person skilled in the art will be aware of standard methods for production of both polyclonal and monoclonal antibodies and fragments thereof which binds to predictive marker, CK2-α in particular. Antibody fragments, particularly Fab fragments and other fragments which retain epitope-binding capacity and specificity are also well known, as are chimeric antibodies, and "humanized" antibodies, in which structural (not determining specificity for antigen) regions of the antibody are replaced with analogous or similar regions from another species. Thus antibodies generated in mice can be "humanized" to reduce negative effects which may occur upon administration to human subjects. Chimeric antibodies are now accepted therapeutic modalities with several now on the market. The present invention therefore comprehends use of antibodies specific for predictive markers, CK2-α in particular, which include F(ab')₂, F(ab)₂, Fab, Fv and Fd antibody fragments, chimeric antibodies in which one or more regions have been replaced by homologous human or non-human portions. The person skilled in the art will also be aware that fragments such as for example sdAb, ScFv fragments and divalent ScFv-type molecules can be prepared using recombinant methods.

The kits can be used for the detection and quantification in immunohistochemical analyses. The antibodies useful for detection may be provided with a label such as a fluorescent or radiolabel.

In the following, the invention will be illustrated by means of the following example as well as the tables and figures.

### FIGURE LEGENDS

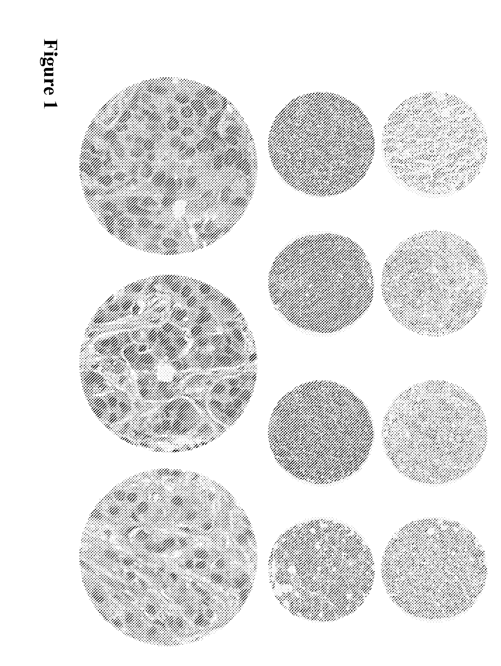

**Figure 1** - TMA cores of node negative breast carcinomas. Immunostaining is predominantly observed in nuclei and to a less extent in cytoplasm. In positive tumors the positive immunoreactivity concerns a variable number of tumor cells and the staining intensity ranges from weak to strong (with various values of mean optical density measured by image analyzer).

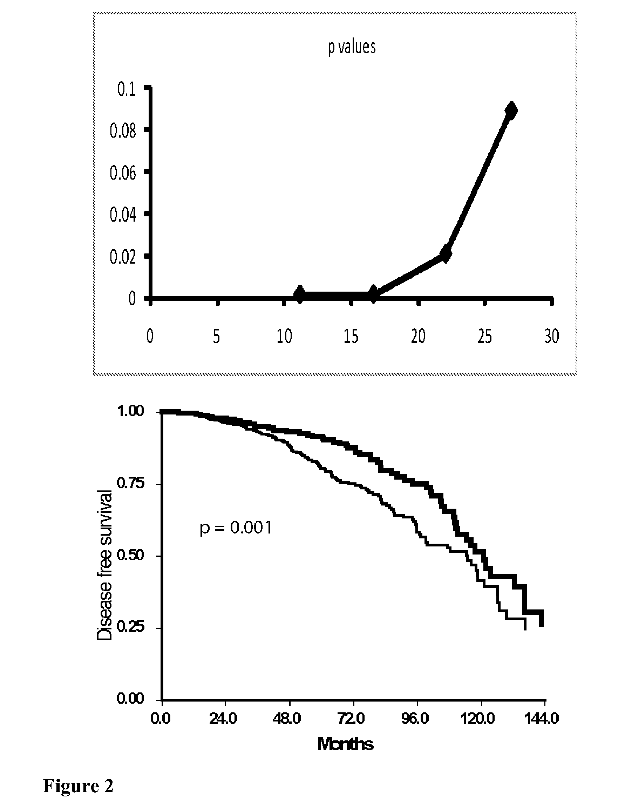

**Figure 2** (Upper panel) The p value curve indicates the relevance and reliability of the threshold used to compared subsets with low and high CK2α expression. ¹⁷ Lower panel on disease-free survival (DFS). Median DFS in patients with high CK2α was x months, compared with y months in patients with low CK2α (p=0.001).

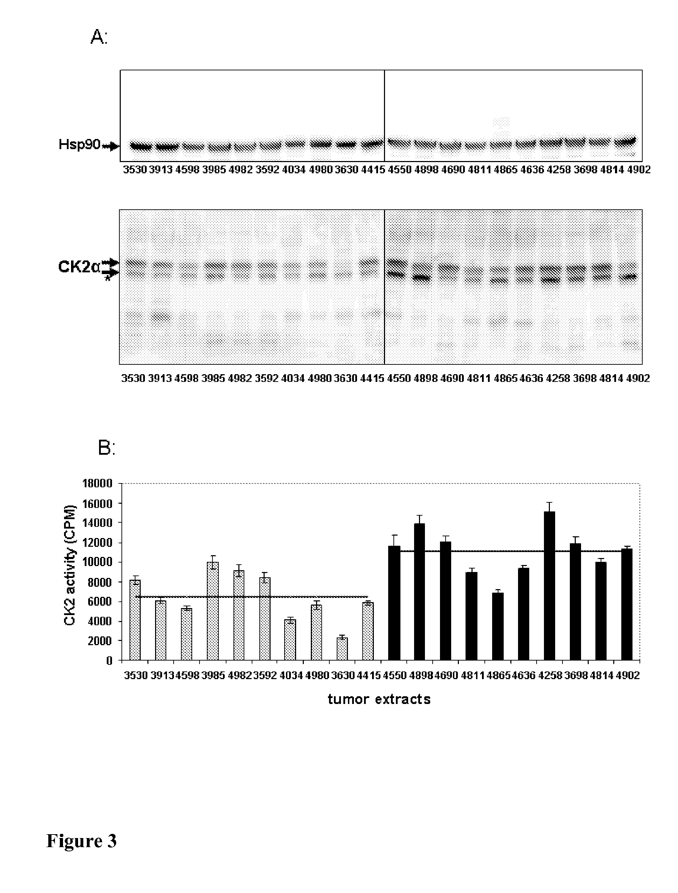

**Figure 3** - CK2α expression and CK2 activity in 20 selected tumor samples. Extracts from 20 selected tumor samples exhibiting low or high IHC scores were analyzed for CK2α expression by western blot and hsp90 was used as a loading control (upper panel). CK2 activity was determined in the same samples that were divided in two groups corresponding to low and high s scores (grey and black respectively). Results are shown as the mean of assays run in triplicate with the error bars representing the standard deviation with a significance of p < 0.01.

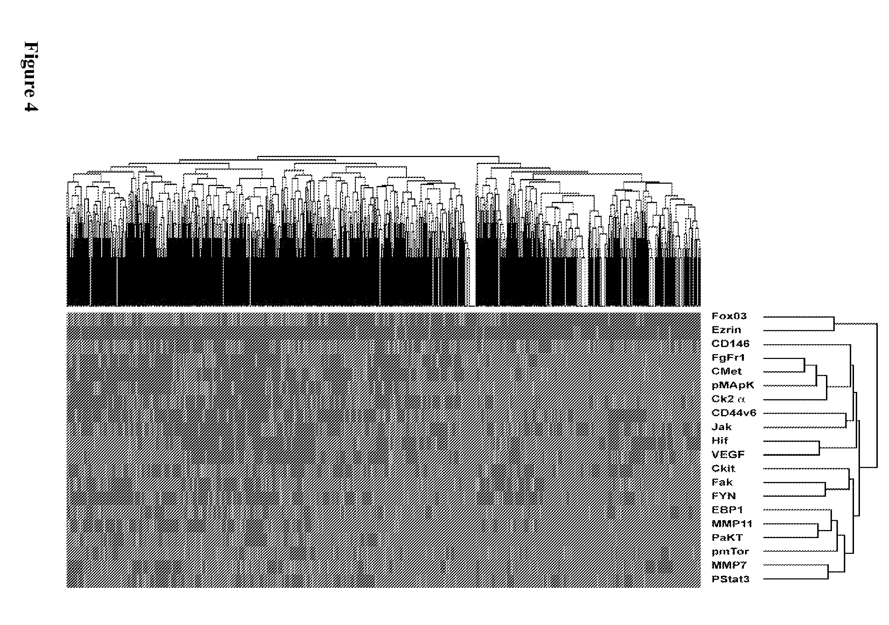

**Figure 4** - Supervised hierarchical clustering of the markers with prognostic significance (metastatic risk) in the log rank test, established with quantitative densitometry of immunohistochemical assays on TMA (n = 572 patients with node-negative breast carcinomas) and quantitative score cut-points for each marker, as determined by log rank test : Each column represents a patient. Each row represents a marker staining as indicated on the right side. Green color represents weak marker staining, red represents strong marker staining. This analysis separated patients into two groups and dendrograms show the close or distant relationships of markers acting on metastasis development and on patients' outcome.

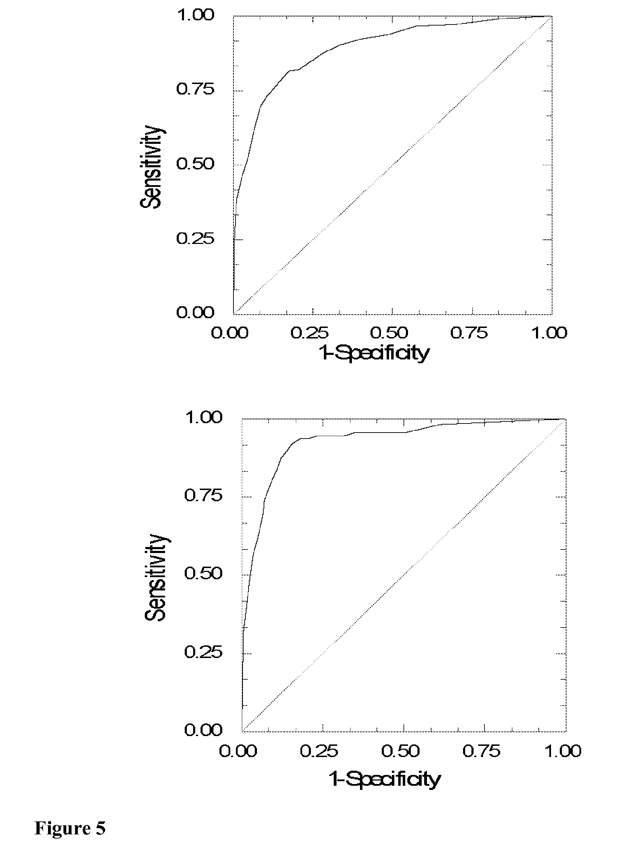

**Figure 5** **-** Logistic regression and ROC curves determined the immunohistochemical signature (SHARP2, STAT1, EIF4E, pMAPKAPK-2, pAKT, caveolin, CK2α, VEGF, FGFR-1 and CK2α), that correctly classified patients whatever their node status and also interestingly node-negative patients: 82.32 % with nine markers / top, and 86.01 % with eleven markers (bottom). The larger the surface under the (ROC) curve, the greater the probability of prediction accuracy.

**Table 1**

| **Markers and groups of markers determined by logistic regression, evaluated by quantitative IHC in 905 breast carcinomas** | |
|---|---|
| **Group of markers** | **Immunohistochemical Markers** |
| **Signalling pathways** | PI3K, pAKT, pMAPKAPK-2, pmTOR, P38 MAPKinase, p4^{E}-BP-1, EIF4E, FOXO3a, FAK, JAK, FYN, STAT-1, STAT-3, SHARP-2, P42 MAPKinase |
| **Angiogenesis** | HIF-1α, VEGF, CD146, CD34, FGFR-1, CA-IX |
| **Tumor spreading** | cMet, caveolin, moesin, βcatenin, MMP7, MMP11, cKit, P cadherin |

**Table 2**

| **Antibodies used in 1,000 breast carcinoma TMA, selected according to literature data and our previous study showing the prognostic relevance of corresponding markers (16) (Ventana Benchmark XT automated device, immunoperoxidase)** | | | | |
|---|---|---|---|---|
| | ***ANTIBODIES*** | **SUPPLIER** | **SOURCE*** | CLONE |
| 1 | MMP7 | Abcam | Rpab | |
| 2 | MMP11 | Abcam | Rmab | EP1259Y |
| 3 | EIF4E | Cell signaling | Rmab | C46H6 |
| 4 | P70 S6 Kinase | Cell signaling | Rmab | 49D7 |
| 5 | FOXO3a | Cell signaling | Rpab | |
| 6 | P 42-MAP-Kinase (ERK-2) | Cell signaling | Rpab | |
| 7 | Phospho-mTOR (Ser2448) | Cell signaling | Rmab | 49F9 |
| 8 | VEGF | R&D Systems | Mmab | 26503 |
| 9 | Phospho-4^{E}-BP-1 (Thr37/46) | Cell signaling | Rmab | 236B4 |
| 10 | HIF-1 α | Gift ** | Mmab | 729T3 |
| 11 | β-Catenin | Novocastra | Mmab | 17C2 |
| 12 | FGFR-1 Flg (C-15) | Santa Cruz | Rpab | |
| 13 | Maspin | BD Pharmingen | Mmab | G167-70 |
| 14 | MET | Chemicon/Abcys | Mmab | 4AT44 |
| 15 | P-Cadherin | Novocastra | Mmab | 56C1 |
| 16 | Ezrin (p81, 80k, cytovillin) | Neomarkers | Mmab | 3C12 |
| 17 | phospho-AKT (Ser473) | Cell Signaling | Rmab | 587F11 |
| 18 | CD 44v6 | Novocastra | Mmab | VFF-7 |
| 19 | **CK2**α | disclosed in WO2008083998 | Rpab | αCOC |
| 20 | Moesin | Neomarkers | Mmab | 38/87 |
| 21 | phospho-STAT-3(Tyr705) | Cell Signaling | Rmab | D3A7 |
| 22 | phospho-MAPKAPK-2 | Cell Signaling | Rmab | (Thr334) |
| 23 | STAT-1 | Cell Signaling | Mmab | 9H2 |
| 24 | FAK | Cell Signaling | Rpab | |
| 25 | P38 MAP-Kinase | Cell Signaling | Rpab | |
| 26 | SHARP 2 | Abcam | Rpab | |
| 27 | FYN | Abcam | Mmab | 1S |
| 28 | Carbonic anhydrase IX | Abcam | Rpab | |
| 29 | CD 117 / KIT | Dako | Rpab | |
| 30 | PI3 Kinase | Cell Signaling | Rpab | |
| 31 | JAK 1 | Cell Signaling | Rpab | |
| 32 | Caveolin 1 | Santa Cruz | Rpab | |
| 33 | CD-146 | Novocastra | Mmab | N1238 |

| | | | | |
|---|---|---|---|---|
| * *Mmab, mouse monoclonal antibody; Rpab, rabbit polyclonal antibody* ** *Kindly provided by Dr J Dr. J. Pouyssegur. CNRS-UMR 6543 (Nice, France).* | | | | |

### EXAMPLE

In the following description, all molecular biology experiments for which no detailed protocol is given are performed according to standard protocol.

### ABSTRACT

**Background**: The aim of this study was to evaluate the prognostic significance of the immunohistochemical expression of CK2α in breast carcinomas.

**Methods**: Quantitative measurements of immunohistochemical expression of 33 biomarkers using high-throughput densitometry, assessed on digitized microscopic tissue micro-array images were correlated with clinical outcome in 1,000 breast carcinomas in univariate and multivariate analysis.

**Results:** In univariate analysis, CK2α was a significant prognostic indicator (p < 0.001). Also, multivariable model allowed the selection of the best combination of the 33 biomarkers to predict patients' outcome through logistic regression. A nine-marker signature highly predictive of metastatic risk, associating SHARP-2, STAT1, EIF4E, pMAPK, pAKT, caveolin, VEGF, FGFR-1 and CK2α permitted to well classify 82.32 % of patients (specificity 81.59 %, sensitivity 92.55 %, area under ROC curve 0.939). Importantly, in node negative subset of patients an even more (86 %) clinically relevant association of eleven markers was found predictive of poor outcome.

**Conclusion:** A strong quantitative CK2α immunohistochemical expression in breast carcinomas is individually a significant indicator of poor prognostic. Moreover, an immunohistochemical signature of 11 markers including CK2α accurately (86 %) well classifies node negative patients in good and poor outcome subsets. Our results indicate that CK2α evaluation together with key downstream CK2 targets would be a useful tool to identify patients at high risk of distant metastases and that CK2 can be considered as a relevant target for potential specific therapy.

In the present study our goal was, using in situ high throughput methods, to investigate CK2α expression by a quantitative immunohistochemical analysis on digitized TMA of a large cohort of breast carcinomas (n = 1,000). This analysis was correlated with overall and metastatic free patients' survival and along with a panel of 32 other biomarkers.

### MATERIALS AND METHODS

### Patients

A consecutive series of 1,000 patients with invasive breast carcinomas, that were operated on from 1998 to 2000 (mean follow-up, 112 months) at Marseille s' *Centre Hospitalo-Universitaire* in Genecology Oncology Departments. Surgery was in all cases the first treatment. For the first step of initial diagnosis and treatment, patients' management was handled by the same group of surgeons and by three senior pathologists (C.Charpin, S.Garcia, S.Giusiano). Conservative treatment, mastectomy and node resection (complete or sentinel) were applied according to current European recommendations. Likewise, radiotherapy, chemotherapy and hormone therapy were applied according to criteria currently used at that time

The 2007 follow-up data showed that 206 were metastatic, and 53 patients deceased.

Analysis of the distribution of the series by age, histological type and grade, and nodal status before TMA construction revealed an usual distribution of breast carcinomas series and no bias in tumor selection, as compared to literature data. Due to technical difficulties in performing immunocytochemical tests on many serial paraffin sections of a TMA to evaluate the 33 different markers, complete data for all markers were finally obtained for only 905 patients out of the initial series of 1,000.

Our study focused mainly on correlation of quantitative immunohistochemical data with patients' outcome. Current histoprognostic criteria on H and E staining were not retained for statistical analysis for two reasons: i) mainly to limit the burden of data, ii) to focus the statistical analysis on continuous variables (numerical values of measurements) homogeneously obtained by densitometric measurements of immunoprecipitates with the image analyzer.

### Immunohistochemistry

### * Tissues

Tumor fragments were sampled large and thick enough to allow further TMA construction. Tissue samples were all taken from surgical specimens after formalin fixation. Attention was paid to optimize consistent tissue-handling procedures, including fast immersion in buffered formalin in an appropriate container by pathologists trained in the procedure. Duration of fixation was 24 h for smaller samples (< 5 cm) and 48 h for larger ones, to improve formalin penetration, before specimen dissection at room temperature. After fixation, paraffin pre-embedding and embedding were performed in currently available automated devices.

Paraffin blocks prepared from 1998-2000 were stored then in the same room, where temperature was controlled and maintained at 20°C prior to TMA construction.

### * TMA construction

The procedure for construction of TMAs was as previously described.¹²⁻¹⁵ Briefly, cores were punched from the selected 1,000 contributive paraffin blocks, distributed in four new blocks including two cores for each tumor (500 cases per block, a total of 2,000 cores) of 0.6mm diameter. In order to avoid false positive staining that might result from stromal or inflammatory cells that could react with the antibodies tested, the tumor areas selected for the TMA punches were dense carcinomatous areas with minimal stroma containing some vessels and few fibroblasts.

All the new TMAs blocks were stored at 4°C and 4 µm thick sections were prepared for each marker to be examined by IHC.

### * Immuuohistochemical procedures

Immunoperoxidase procedure was performed using an automated Ventana Benchmark XT device and Ventana kits on serial tissue sections that were prepared and stored at 4°C 24h before immunohistochemical processing, as previously reported¹²⁻¹⁶.

Markers used were some of those reported in the literature as involved in tumor cell spreading, angiogenesis, and involved in main signalling pathways (Table 1). Markers were detected using commercially documented antibodies (Table 2). The selection also hinged upon our experience of immunostaining quality in pre-tests using commercially available antibodies on frozen tissue and current full paraffin sections, prior to high-throughput immunodetection on TMAs. Dilutions of antibodies were determined by a pre-screening analysis on the usual full paraffin 4µm thick sections prior to use on TMA sections.

Specificity of antibodies was documented by the suppliers, and anti CK2α antibody characteristics were previously described along with a screening of prostate carcinomas.^{11,16} Antibodies recognizing phosphorylated molecules are identified with "p" sign such as pmTOR or pAKT. In contrast those simply indicated like STAT 1, FYN, FAK, recognize non phosphorylated molecules (Table 2).

### * Image analysis

Automated densitometric measurements of immunoprecipitates in cores were assessed for each marker antibody in each core individually identified after digitization and image cropping of the slides, as previously reported.^{12,13,15-17} Briefly, TMA analysis with a SAMBA 2050 automated device (SAMBA / TRIBVN, Châtillon 92320, France)¹⁵⁻¹⁷ was performed according to the following protocol. First, an image of the entire slide was built up using low-power magnification (× 2). This image was made up of a mosaic of images acquired along a rectangular grid with contiguous fields. Second, the area of the slide containing the TMA cores was automatically delineated and scanned at higher magnification (×20, pixel dimension: 7.4 µm). Third, after autofocusing, the images were acquired with an overlap greater than the largest mechanical positioning error. Using the image contents, a matching algorithm determined precisely the relative position of each image with respect to its neighbors. Calculated overlap was removed from images to produce a new set of higher-magnification images, thus covering accurately the cores of interest. A specially developed tool referred to as TMA crop then allowed superposition of the TMA grid onto the reduced image and precise alignment of each node of the grid with the core location within the image. The final step was performed automatically using the core image contents to ensure pixel precision of the match. From the images acquired with ×20 magnification, a new set of images was next computed, one for each core. For color analysis of the core images, the SAMBA appropriate software was applied as previously reported^{12,13} in usual full tissue sections. In the present study, we correlated the patients' follow-up parameters with a quantitative score combining the surface stained and the intensity of staining¹⁵⁻¹⁷ computed by the SAMBA "immuno-software".

### * Statistical analysis

Immunohistochemical expression of each marker was first correlated with patients' disease-free survival using NCSS and Statistica statistical softwares.

The prognostic significance was in univariate analysis determined by log rank tests (Kaplan-Meier curves). The appropriate threshold of prognostic significance for a given marker was determined as previously described¹⁷ and recommended^{15,16} for univariate analysis.

Supervised hierarchical clustering of significant prognostic indicators in the series provided qualitative data to be compared through with previously reported research results documenting the relationship and the role played by these molecules in the process of cancer metastasis. ^{13,15,16}

Importantly, we used logistic regression and ROC curves to identify the combination of markers (signature) with the best sensitivity and specificity for prognosis prediction. Multivariable fractional polynomials model of logistic regression was used to correlate the outcome variable with the quantitative IHC expression of markers quantitatively evaluated as continuous variables without predetermined cut-points.^{18,19}

### Western blot analysis

Protein extracts were prepared by homogenizing frozen powdered breast tumors in RIPA buffer containing a cocktail of protease and phosphatase inhibitors (50 mM Tris-HCl, pH 7.5, 1% Triton X-100, 150 mM NaCl, 1 mM NaF, 1 mM Na₃VO₄, 10 mM sodium pyrophosphate, 1 mM phenylmethylsulfonyl fluoride, 1 µg/ml aprotinin, 1 µg/ml leupeptin), followed by centrifugation at 14,000 x g to remove debris. Western blotting was performed according to conventional protocols. Briefly, samples of tumor extracts (20 µg protein) were separated by SDS-PAGE on 4-12% acrylamide-gradient gel and transferred on nitrocellulose. The membranes were blocked in PBS containing 5% milk and 0.05% Tween-20 and incubated overnight with CK2α subunit specific antibodies (CK2α COC 1/1000). A goat anti-rabbit antibody coupled to horseradish peroxidase was used at a dilution of 1: 10,000. Antigens were visualized using enhanced chemiluminecence (Perkin Elmer) and Fusion FX acquisition systems. Verification of equal loading was done by immunodetection of HSP90 (Stressgen clone13F1 1/1000).

### CK2 kinase activity

CK2 kinase assays were performed in a final assay volume of 18 µl containing 3µl of tumor extract (3µg protein) and a mixture containing 167µM of peptide substrate (RRREDEESDDEE (SEQ ID NO:2), 20mM MgCl₂, and 1µCi of (γ-³²P) ATP (6000 Ci/mmol). The final concentration of ATP was 25 µM. Assays were performed under linear kinetic conditions for 5 min at 22°C before termination by the addition of 60 µl of 4% trichloroacetic acid.

### RESULTS

### Distribution of positive CK2α staining within tumor cells

Immunohistochemical analysis of individual slides showed that CK2α staining was observed both in cell nucleus and cytoplasm (Fig.1) Measurements of this pancellular distribution of CK2α were assessed on digitized microscopic images enclosing all spots in individual slide. After "cropping" of the images, densitometry was assessed by the analyser in each spot identified using a software specifically developed for immunohistochemically stained sections. The degree and extent of immunostaining were automatically evaluated and use to compute quantitative scores (QS) consequently defined by the analyser software.

### Univariate analysis

On the basis of literature data on breast carcinoma biomarkers enrolled in metastatic and angiogenic processes together with molecules of major signalling pathways of tumor cell machinery, 32 biomarkers were selected (Table 1) and screened along with the expression of CK2α in TMAs containing tumors from disease-free patients and from patients with metastasis and recurrent disease.

The first step in assessing prognostic value consisted in comparison of mean quantitative scores in relation to the number of positive and negative patients in the disease-free and diseased categories (Mann-Whitney and chi-squared tests). Mean CK2α quantitative scores in patients with metastasis, were significantly greater than those with disease free survival. The prognostic significance of markers was further individually evaluated by a univariate log rank test (Kaplan-Meier survival curves). There was a significant difference (p< 0.001) in median DFS between low and high CK2α-expressing patients (Fig.2 lower panel).

The threshold of staining of prognostic significance was first determined according to the p value curves from univariate analysis (log rank), as reported by Altman et al.¹⁷ and previously used¹³⁻¹⁶ as shown for CK2α (Fig.2 upper panel) (p < 0.001).

To validate the IHC analysis, we performed a biochemical analysis of CK2α expression and activity on a selected subset of 20 tumor samples exhibiting a wide range of IHC scores (1 to 84). Western blotting analysis was done to determine the level of CK2α catalytic protein. As shown in Fig.3, CK2α expression in tumor samples correlated with the quantitative immunohistochemical scores. Similarly, CK2 kinase activity paralleled with the CK2α expression level indicating that the increase in CK2 activity might be largely attributable to upregulation of CK2α expression. Thus, this biochemical analysis validates our quantitative immunostaining analysis.

Fig.4 illustrates in supervised hierarchical clustering the relationship between CK2α and significant prognostic markers in the log rank test. This method separated patients into two groups illustrating the respective distribution of markers with regards to the metastatic clinical events. Dendograms, established according to the expression of individual markers with regard to favorable or poor outcome patients, show the close relationships between groups of markers, suggesting that they may play similar roles in metastasis development that impact on patients'outcome. Importantly, cells from the patient group with the highest risk of metastasis stained positive for all markers tested. Thus, this combination of multiple markers strongly correlates with clinicopathological parameters.

### Multivariate analysis

### • Logistic regression (ROC curves)

The relationship between groups of predictive markers and the patients' outcome variable was then evaluated by the multivariable fractional polynomials model of logistic regression.^{18,19} In this method no cut-point was predetermined for regression and marker immunostaining values were considered as continuous variables.

The optimal combination computerized from the image analysis data bank for the markers in the series of breast carcinomas is shown in Table 2.

When CK2α was associated with markers of main signalling pathways of angiogenesis and of tumor metastatic progression, (table 1) the multivariable fractional polynomials regression showed that CK2α was included in an optimal combination of nine markers predictive of high metastatic risk. The analysis shown in Fig.5 well classified patients in this category of poor prognosis (lower panel) in 82.32 % of the cases (specificity 81.5 %, 168/206 ; sensitivity 577/699, 82.55 %) area under ROC/receiving operating characteristic curve = 0.939). This combination included SHARP2, STAT1, EIF4E, pAKT, caveolin, CK2α, pMAPK, VEGF, FGFR-1, in which SHARP2 ranked as first, FGFR-1 as ninth, and CK2α as sixth.

According to the estimated regression model, the probability of metastasis was computed from quantitative scores obtained after densitometry measurements for each marker, and with the following logit = 0.6156 + 1.9654E-02.Cav + 0.2301E-02.CK2α + 0.04850E-02.EIF4E + 5.9821E-04.FGFR-1 + 9.0567E-03.pAKT + 3.1445E-03.pMAPK + 4.59525E-03.SHARP2 + 0.13057E-03.STAT1 +8.23921E-03.VEGF).

This model estimates "B" for a specific group (in the present analysis the group of patients with metastases) where logit (Y) = X B, and X = densitometric quantitative score for each marker.

To calculate the probability of classifying in the correct category of outcome, the logit is transformed using Prob = exp (- logit) / [1 + exp (- logit)] or Prob = exp (- XB) / [1 + exp (-XB)].

Interestingly, in node negative patients' subset (n = 572), CK2α was also included in another optimal combination of 11 markers, well classifying patients in 86.01 % (sensitivity 102/111, 91.89 % and specificity 390/461, 84.6 % area under ROC curve = 0.928).In this combination HIF-1α, SHARP2, EIF4E, pMAPK, MMP7, pAKT, CK2α, FGFR-1, pmTOR, Fyn and JAK. HIF-1α ranked as first, and CK2α as seventh and JAK as last, with a logit = 3.8297E-02 + 2.2727E-03.CK2α + 6.3130E-03. EIF4E + 7.1550E-03.FGFR1 + 2.2779.FYN + .18505E-02.HIF-1α + .6530-03.JAK + 6.1954E-03.MMP7 + .12559E-02.pAKT + 7.5238E-03.pMAPK + 24276E-03.pmTOR + 5.9089E-03.SHARP2.

### • Validation sets

Validation sets were established for the 905 series with one third (n = 302) and two thirds (n= 603) of the total number of cases showing similar results, with 80.5 % and 83.7 % of patients correctly classified respectively.

For node negative set, well-classified patients accounted for 83.2 % and 84.3 % for the one third and two thirds of the entire 572 patients' set, respectively.

### Spearmean's correlation coefficient

In an attempt to better document CK2α interactions with other markers in breast carcinoma metastatic process, Spearman's correlation coefficient was computed according to the localization of markers on dendrograms (Fig. 4) and to literature data.

A significant (p < 0.001) correlation was observed between CK2α and PTEN (p = 0.55), and pAKT (p = 0.53), whereas Spearman's coefficient was weaker (p < 0.001) with PI3K (p = 0.37), E-cadherin (p = 0.31), β catenin (p = 0.26), and even weaker with HIF-1α (p = 0.13) and with other markers (p < 0.15), individually.

### DISCUSSION

The classification of breast cancers into subgroups on the basis of gene expression patterns is regarded as a method of choice, but widespread use of gene-expression profiling in clinical setting remains limited. Consequently, there is interest in using immunohistochemical markers to classify breast cancer into subtypes that are biologically distinct and behave differently. The aim of this study was to evaluate the pronostic significance of CK2α expression in association with various clinicopathological parameters in 905 patients with breast tumors. Elevated CK2 activity in human breast tumor specimens has been reported.¹⁰ However, clinical data dealing with the specific expression of CK2α at the protein level are scarce. Consequently, the pronostic value of CK2α in breast cancer remains uncertain. With a cohort of 905 breast tumor specimens, the scope of our study is consistent. Using high-throughput densitometry of digitized microscopic tissue micro-array images, quantitative measurements of IHC expression of 33 biomarkers were correlated with clinical outcome in univariate analysis and with logistic regression. Our study demonstrates for the first time in breast cancer, a strong association between CK2α overexpression and tumor aggressiveness suggesting that this kinase could be an adverse prognostic marker. Moreover, when CK2α was associated with markers of main signalling pathways, involved in angiogenesis and tumor metastatic progression, a multivariable analysis showed that CK2α ranked favorably in an optimal combination of nine markers predictive of high metastatic risk. Collectively, our data support the notion that high CK2α allows an accurate classification of patients in this category of poor prognosis. Indeed, deregulated expression of CK2 in cells can be oncogenic as transgenic expression of CK2α can promote mammary gland tumorogenesis.¹⁰ However, the mechanistic basis of CK2α dysregulation is currently unclear. CK2α plays a key role in promoting cell survival under stress conditions. Therefore, its overexpression in breast cancer cells might not only be related to the increased proliferative capacity of dedifferentiated tumor cells suggesting that CK2 activity is required for ongoing tumor growth, but also for their marked resistance to apoptotic signals. CK2 activity has been implicated in transcriptional regulation, differentiation and development, DNA damage signalling and cell survival.^{1,20,21} This broad range of cellular response is orchestrated through CK2-dependent regulation of key signalling molecules in the PI3K/AKT/mTOR, NF_{K}B, Wnt and hypoxia pathways.

For example, it was demonstrated that primary breast cancer samples from patients or from carcinogen-induced rodent models as well as breast cancer cell lines display increased CK2 activity and aberrant activation of NF_{K}B which in turn, modulates the survival and the transformed phenotype of breast cancers.¹⁰ Moreover, CK2 also participates in Wnt/β-catenin signalling in mammary epithelial cells.²²

Our analysis shows a positive relationship between CK2α and a group of markers predictive of patients' outcome, such as SHARP2, STAT1, EIF4E, pAKT, caveolin, pMAPK, VEGF, FGFR-1. Several components of the PTEN/PI3K/AKT signalling pathway that are involved in cell survival have been described as causal forces in cancer and breast cancers are particularly reliant on this signalling pathway. Interestingly, CK2 acts as a «lateral» regulator in the PI3K/AKT/mTOR transduction pathway.²¹ For example, the tumor suppressor PTEN is a phosphatidylinositol D3-phosphatase that acts as a negative regulator of this pro-survival pathway. Multiple kinases including CK2 target PTEN providing a negative regulation of its activity and thereby triggering a robust activation of the PI3K/AKT pathway.^{23,24} Dephosphorylation and inactivation of AKT is counteracted by CK2, reinforcing the activation of this survival pathway.^{25,26} Altogether, these observations are in accordance with the significant correlation that we observed between CK2α, PTEN and pAKT assessed by Spearman correlation analysis. Activation of signal transducers and activators of transcription (STATs) plays an important role in the maintenance of growth and differentiation. Constitutively activated STATs facilitate neoplastic behaviors of a variety of cancers. It was reported that STAT1 is a downstream target of CK2 and Serine-phosphorylated STAT1 plays a critical role in the pathogenesis of Wilms' tumor and possibly other neoplasms with similar STAT1 phosphorylation patterns.²⁷

A potential functional link between CK2 and translational initiation/repression has been described. CK2 phosphorylates the proline-rich homeodomain protein (PRH/Hex) that regulates mRNA transport by binding to translational initiation factor EIF4E.²⁸

HIF-1α and VEGF are mechanistically linked to intratumor hypoxia and play important role in angiogenesis and tumor progression.²⁹ Indeed, it has been observed that CK2 activity is increased in hypoxic conditions and participates to the upregulation of HIF-1α transactivation activity.³⁰ Since intratumor hypoxia increases the tumorigenicity of cancer cells by selecting more aggressive and metastatic clones, overexpression of CK2 in breast cancer could participate to the molecular circuits that propagate the cancer phenotype through the HIF-1α/VEGF interplay.

Many individual molecular prognostic factors have been identified in patients with primary breast cancer, but few have individually played a significant role in disease management, whereas a multiple marker approach identifying active signalling pathways has begun to guide the prediction of prognosis or treatment response.

In accordance, our data support the notion that CK2α has a clear biological relevance for the prognosis in node-negative breast carcinoma. The incorporation of CK2α into prognostic tools currently used in clinical practice would be useful to reliably identify patients at high-risk of disease recurrence, and distant metastasis development.

### References

Throughout this application, various references describe the state of the art to which this invention pertains. The disclosures of these references are hereby incorporated by reference into the present disclosure.
1. Litchfield DW. Protein kinase CK2: structure, regulation and role in cellular decisions of life and death. Biochem J 2003;369:1-15.
2. St-Denis NA, Litchfield DW. Protein kinase CK2 in health and disease: From birth to death: the role of protein kinase CK2 in the regulation of cell proliferation and survival. Cell Mol Life Sci 2009;66:1817-29.
3. Filhol O, Cochet C. Protein kinase CK2 in health and disease: Cellular functions of protein kinase CK2: a dynamic affair. Cell Mol Life Sci 2009;66:1830-9.
4. Duncan JS, Litchfield DW. Too much of a good thing: the role of protein kinase CK2 in tumorigenesis and prospects for therapeutic inhibition of CK2. Biochim Biophys Acta 2008;1784:33-47.
5. Wang G, Ahmad KA, Ahmed K. Role of protein kinase CK2 in the regulation of tumor necrosis factor-related apoptosis inducing ligand-induced apoptosis in prostate cancer cells. Cancer Res 2006;66:2242-9.
6. Pallares J, Llobet D, Santacana M, et al. CK2beta is expressed in endometrial carcinoma and has a role in apoptosis resistance and cell proliferation. Am J Pathol 2009;174:287-96.
7. Laudet B, Barette C, Dulery V, et al. Structure-based design of small peptide inhibitors of protein kinase CK2 subunit interaction. Biochem J 2007;408:363-73.
8. Yde CW, Frogne T, Lykkesfeldt AE, Fichtner I, Issinger OG, Stenvang J. Induction of cell death in antiestrogen resistant human breast cancer cells by the protein kinase CK2 inhibitor DMAT. Cancer Lett 2007;256:229-37.
9. Zhu D, Hensel J, Hilgraf R, et al. Inhibition of protein kinase CK2 expression and activity blocks tumor cell growth. Mol Cell Biochem 2010;333:159-67.
10. Landesman-Bollag E, Romieu-Mourez R, Song DH, Sonenshein GE, Cardiff RD, Seldin DC. Protein kinase CK2 in mammary gland tumorigenesis. Oncogene 2001;20:3247-57.
11. Laramas M, Pasquier D, Filhol O, Ringeisen F, Descotes JL, Cochet C. Nuclear localization of protein kinase CK2 catalytic subunit (CK2alpha) is associated with poor prognostic factors in human prostate cancer. Eur J Cancer 2007;43:928-34.
12. Charpin C, Vielh P, Duffaud F, et al. Quantitative immunocytochemical assays of P-glycoprotein in breast carcinomas: correlation to messenger RNA expression and to immunohistochemical prognostic indicators. JNatl Cancer Inst 1994;86:1539-45.
13. Garcia S, Dales JP, Jacquemier J, et al. c-Met overexpression in inflammatory breast carcinomas: automated quantification on tissue microarrays. Br J Cancer 2007;96:329-35.
14. Makretsov NA, Huntsman DG, Nielsen TO, et al. Hierarchical clustering analysis of tissue microarray immunostaining data identifies prognostically significant groups of breast carcinoma. Clin Cancer Res 2004;10:6143-51.
15. Garcia S, Dales JP, Charafe-Jauffret E, et al. Poor prognosis in breast carcinomas correlates with increased expression of targetable CD146 and c-Met and with proteomic basal-like phenotype. Hum Pathol 2007;38:830-41.
16. Charpin C, Secq V, Giusiano S, et al. A signature predictive of disease outcome in breast carcinomas, identified by quantitative immunocytochemical assays. Int J Cancer 2009;124:2124-34.
17. Altman DG, Lausen B, Sauerbrei W, Schumacher M. Dangers of using "optimal" cutpoints in the evaluation of prognostic factors. J Natl Cancer Inst 1994;86:829-35.
18. Royston P, Altman DG, Sauerbrei W. Dichotomizing continuous predictors in multiple regression: a bad idea. Stat Med 2006;25:127-41.
19. von Elm E, Altman DG, Egger M, Pocock SJ, Gotzsche PC, Vandenbroucke JP. The Strengthening the Reporting of Observational Studies in Epidemiology (STROBE) statement: guidelines for reporting observational studies. Lancet 2007;370:1453-7.
20. Filhol O, Baudier J, Delphin C, Loue-Mackenbach P, Chambaz EM, Cochet C. Casein kinase II and the tumor suppressor protein P53 associate in a molecular complex that is negatively regulated upon P53 phosphorylation. J Biol Chem 1992;267:20577-83.
21. Ruzzene M, Pinna LA. Addiction to protein kinase CK2: a common denominator of diverse cancer cells? Biochim Biophys Acta 2010;1804:499-504.
22. Ji H, Wang J, Nika H, et al. EGF-induced ERK activation promotes CK2-mediated disassociation of alpha-Catenin from beta-Catenin and transactivation of beta-Catenin. Mol Cell 2009;36:547-59.
23. Ning K, Miller LC, Laidlaw HA, et al. Leptin-dependent phosphorylation of PTEN mediates actin restructuring and activation of ATP-sensitive K+ channels. J Biol Chem 2009;284:9331-40.
24. Silva A, Jotta PY, Silveira AB, et al. Regulation of PTEN by CK2 and Notch1 in primary T-cell acute lymphoblastic leukemia: rationale for combined use of CK2- and gamma-secretase inhibitors. Haematologica;95:674-8.
25. Di Maira G, Brustolon F, Pinna LA, Ruzzene M. Dephosphorylation and inactivation of Akt/PKB is counteracted by protein kinase CK2 in HEK 293T cells. Cell Mol Life Sci 2009;66:3363-73.
26. Olsten ME, Canton DA, Zhang C, Walton PA, Litchfield DW. The Pleckstrin homology domain of CK2 interacting protein-1 is required for interactions and recruitment of protein kinase CK2 to the plasma membrane. J Biol Chem 2004;279:42114-27.
27. Timofeeva OA, Plisov S, Evseev AA, et al. Serine-phosphorylated STAT1 is a prosurvival factor in Wilms' tumor pathogenesis. Oncogene 2006;25:7555-64.
28. Soufi A, Noy P, Buckle M, Sawasdichai A, Gaston K, Jayaraman PS. CK2 phosphorylation of the PRH/Hex homeodomain functions as a reversible switch for DNA binding. Nucleic Acids Res 2009;37:3288-300.
29. Kallergi G, Markomanolaki H, Giannoukaraki V, et al. Hypoxia-inducible factor-1 alpha and vascular endothelial growth factor expression in circulating tumor cells of breast cancer patients. Breast Cancer Res 2009;11:R84.
30. Mottet D, Ruys SP, Demazy C, Raes M, Michiels C. Role for casein kinase 2 in the regulation of HIF-1 activity. Int J Cancer 2005;117:764-74.

## Claims

1. A method of determining the prognosis of a patient suffering from breast cancer, comprising the step of measuring the level of expression of the CK2α subunit in breast cancer cells obtained from said patient, wherein a high CK2α expression is associated with a poor prognosis.

2. The method according to claim 1, wherein the CK2α expression is measured with an antibody or a fragment thereof which specifically binds to a CK2-α epitope as set forth in SEQ ID NO:1.

3. The method according to claim 1 or 2, wherein said breast cancer cells obtained from said patient originate from a surgical act of tumour resection or from a biopsy.

4. The method according to any of claims 1 to 3, wherein the patient suffering from breast cancer is a node-negative patient.

5. The method according to any of claims 1 to 4, wherein the levels of expression of 1, 2, 3 or 4 predictive markers are quantified together with the expression of CK2α, and wherein the predictive markers are selected from the group consisting of SHARP-2, EIF4E, pMAPK, and pAKT.

6. The method according to claim 5, wherein the levels of expression of 4 predictive markers are quantified together with the expression of CK2α, and wherein the predictive markers are SHARP-2, EIF4E, pMAPK, and pAKT.

7. The method according to any of claims 1 to 4, wherein the levels of expression of 1, 2, 3, 4, 5, 6, 7, or 8 predictive markers are quantified together with the expression of CK2α, and wherein the predictive markers are selected from the group consisting of SHARP2, STAT1, EIF4E, pAKT, caveolin, pMAPK, VEGF and FGFR-1.

8. The method according to claim 7, wherein the levels of expression of 8 predictive markers are quantified together with the expression of CK2α, and wherein the predictive markers are SHARP2, STAT1, EIF4E, pAKT, caveolin, pMAPK, VEGF and FGFR-1.

9. The method according to claim 4, wherein the levels of expression of 1, 2, 3, 4, 5, 6, 7, 8, 9, or 10 predictive markers are quantified in a node-negative patient together with the expression of CK2α, and wherein the predictive markers are selected from the group consisting of HIF-1α, SHARP2, EIF4E, pMAPK, MMP7, pAKT, FGFR-1, pmTOR, Fyn and JAK.

10. The method according to claim 4, wherein the levels of expression of 10 predictive markers are quantified in a node-negative patient together with the expression of CK2α, and wherein the predictive markers are HIF-1α, SHARP2, EIF4E, pMAPK, MMP7, pAKT, FGFR-1, pmTOR, Fyn and JAK.

11. A kit comprising:
a) a binding partner capable of selectively interacting with CK2-α ; and
b) 1, 2, 3, or 4 different binding partners which specifically bind to 1, 2, 3 or 4 respectively, predictive markers selected from the group consisting of SHARP-2, EIF4E, pMAPK, and pAKT.

12. The kit of claim 11, wherein the kit comprises 4 different binding partners which specifically bind to SHARP-2, EIF4E, pMAPK, and pAKT respectively.

13. A kit comprising:
a) a binding partner capable of selectively interacting with CK2-α ; and
b) 1, 2, 3, 4, 5, 6, 7, or 8 different binding partners which specifically bind to 1, 2, 3, 4, 5, 6, 7, or 8 respectively, predictive markers selected from the group consisting of SHARP2, STAT1, EIF4E, pAKT, caveolin, pMAPK, VEGF and FGFR-1.

14. A kit comprising:
a) a binding partner capable of selectively interacting with CK2-α ; and
b) 1, 2, 3, 4, 5, 6, 7, 8, 9, or 10 different binding partners which specifically bind to 1, 2, 3, 4, 5, 6, 7, 8, 9, or 10 respectively, predictive markers selected from the group consisting of HIF-1α, SHARP2, EIF4E, pMAPK, MMP7, pAKT, FGFR-1, pmTOR, Fyn and JAK.

15. A kit according to any of claims 10 to 14, wherein the binding partner capable of selectively interacting with CK2-α is an antibody or a fragment thereof which specifically binds to a CK2-α epitope as set forth in SEQ ID NO: 1.
